# EUROPEAN PATENT APPLICATION

(11) **EP 2 341 150 A1**
(43) Date of publication of application: **06.07.2011**
(21) Application number: 09814641.8
(22) Date of filing: 17.09.2009
(51) Int. Cl.: C12Q 1/68, C12N 15/09

(54) **BREAST CANCER METASTASIS DETERMINATION METHOD AND BLOOD SERUM EVALUATION METHOD**

(30) Priority: 19.09.2008 JP 2008241302
(71) Applicant: Sysmex Corporation, Kobe-shi, Hyogo 651-0073 (JP)
(72) Inventor: YAMAMOTO,Noriaki, Kobe-shi Hyogo 651-0073 (JP); KAJITA,Masahiro, Kobe-shi Hyogo 651-0073 (JP); SAKAI,Ayako, Kobe-shi Hyogo 651-0073 (JP)
(74) Representative: Paemen, Liesbet R.J.
(86) International application number: PCT/JP2009/066278
(87) International publication number: WO 2010/032797

(57) **Abstract**

It is determined that the breast cancer has metastasized when methylation is detected in the CpG island of at least one selected from the group consisting of GSTP1, RASSF1A and RARb by detecting methylation in CpG island of each of GSTP1, RASSF1A and RARb contained in serum obtained from a patient who has undergone surgery to remove breast cancer.

## Description

### TECHNICAL FIELD

The present invention relates to a method for determining breast cancer metastasis which determines the presence or absence of breast cancer metastasis in a patient who has undergone a surgery to remove breast cancer and a method for evaluating serum useful to obtain an indicator for determining breast cancer metastasis.
This application claims the priority of Japanese Patent Application No. 2008-241302, filed on September 19, 2008, the entire contents of which are hereby incorporated by reference.

### BACKGROUND ART

In a patient who has undergone a surgery to remove breast cancer, confirmation of the presence or absence of breast cancer metastasis is important to decide on courses of treatment after the surgery. Particularly, when distant metastasis in which cancer metastasizes to the organs and the lymph nodes far from a primary lesion is found, the confirmation of the presence or absence of breast cancer metastasis is very important to provide a patient with an appropriate care.

As a technique for diagnosing a recurrence of cancer by breast cancer metastasis after the surgery, imaging tests such as echo, MRI and mammography are known. However, there is a disadvantage such that proficient skills are required for these imaging tests, and that apparatuses used in the imaging tests may give a patient an uncomfortable feeling and also the cost thereof is high.

In chromosomal DNA of a higher eukaryote such as human, cytosine in a CpG site comprising a CG dinucleotide sequence is methylated in some cases. This methylation of cytosine in a CpG site functions as a mechanism for suppressing expression of genes. For example, a region which is rich in CpG sites is present in a promoter region of a gene and on/off of transcription from DNA of the gene is controlled by the presence or absence of methylation of cytosine in this promoter region of a gene.

Control of gene expression by DNA methylation plays an important role in events such as early embryo development, tissue-specific gene expression, gene imprinting, inactivation of X chromosome, stabilization of chromosome and typing of DNA replication. In addition, it has been reported that DNA methylation may be highly involved in diseases such as cancer.

As a method for analyzing the above DNA methylation, the methylation-specific PCR method is known. In this method, a treatment of converting cytosine that is not methylated (unmethylated cytosine) in DNA to be analyzed into another base (unmethylated cytosine conversion treatment) is carried out by using bisulfite that is a reagent for converting unmethylated cytosine into another base (unmethylated cytosine conversion agent). Moreover, the presence or absence of DNA methylation can be detected by performing PCR reaction with a primer set for amplifying a sequence in a case where cytosine is not converted into another base, and then examining the presence or absence of the amplification product.

Here, GSTP1, RASSF1A and RARb are genes in which the CpG island is known to be highly methylated in a primary lesion of breast cancer. In addition, Non-Patent Literature 1 describes a method for screening for breast cancer by examining the presence or absence of methylation in the CpG islands of APC, GSTP1, RASSF1A and RARb, contained in serum collected from a patient. However, Non-Patent Literature 1 does not describe anything about determination of the presence or absence of breast cancer metastasis in a patient after surgery.

### PRIOR ART DOCUMENT

### NON-PATENT LITERATURE

Non-Patent Literature 1: Mohammad O. Hoque et al., "Detection of Aberrant Methylation of Four Genes in Plasma DNA for the Detection of Breast Cancer," Journal of Clinical Oncology, Vol. 24, No. 26, September 10, 2006, p. 4262-4269

### SUMMARY OF INVENTION

An object of the present invention is to provide a method for determining breast cancer metastasis which can easily determine the presence or absence of breast cancer metastasis in a patient who has undergone a surgery to remove breast cancer at low cost without giving the patient an uncomfortable feeling. In addition, an object of the present invention is to provide a method for evaluating serum which can obtain a useful indicator for determining breast cancer metastasis which can easily obtain an indicator for determining the presence or absence of breast cancer metastasis in a patient who has undergone a surgery to remove breast cancer at low cost without giving the patient an uncomfortable feeling.

That is, the present invention relates to:
(1) a method for determining breast cancer metastasis comprising the steps of:
   obtaining serum from a patient who has undergone surgery to remove breast cancer,
   detecting methylation in CpG island of each of GSTP1, RASSF1A and RARb contained in the serum obtained in the step of obtaining, and
   determining that the breast cancer has metastasized, when methylation is detected in the CpG island of at least one selected from the group consisting of GSTP1, RASSF1A and RARb in the step of detecting;
(2) the method according to (1), wherein the metastasis is distant metastasis;
(3) the method according to (1), wherein the methylation in CpG island of each of GSTP1, RASSF1A and RARb is detected by using a methylation-specific PCR method in the step of detecting;
(4) the method according to (1), wherein the methylation-specific PCR method is performed by using a first primer set comprising the primers shown in SEQ ID NO: 1 and SEQ ID NO: 2,
   a second primer set comprising the primers shown in SEQ ID NO: 3 and SEQ ID NO: 4, and
   a third primer set comprising the primers shown in SEQ ID NO: 5 and SEQ ID NO: 6;
(5) a method for determining breast cancer metastasis comprising the steps of:
   obtaining serum from a patient who has undergone surgery to remove breast cancer,
   detecting methylation in CpG island of each of GSTP1, RASSF1A and RARb contained in the serum obtained in the step of obtaining,
   measuring an amount of tumor marker for breast cancer contained in the serum obtained in the step of obtaining, and
   determining that the breast cancer has metastasized, when methylation is detected in the CpG island of at least one selected from the group consisting of GSTP1, RASSF1A and RARb in the step of detecting and the amount of the tumor marker obtained in the step of measuring is an abnormal amount;
(6) the method according to (5), wherein the tumor marker for breast cancer is CEA and/or CA15-3;
(7) a method for evaluating serum comprising the steps of:
   (A) determining the presence or absence of methylation in CpG island of each of GSTP1, RASSF1A and RARb contained in serum collected from a patient who has undergone surgery to remove breast cancer, and
   (B) evaluating the serum as serum collected from a patient whose breast cancer has metastasized, when methylation is detected in the CpG island of at least one selected from the group consisting of GSTP1, RASSF1A and RARb contained in the serum in the step (A); and
(8) a method for evaluating serum comprising the steps of:
   (a) determining the presence or absence of methylation in CpG island of each of GSTP1, RASSF1A and RARb contained in serum collected from a patient who has undergone surgery to remove breast cancer,
   (b) measuring an amount of tumor marker for breast cancer contained in the serum, and
   (c) evaluating the serum as serum collected from a patient whose breast cancer has metastasized, when methylation is detected in the CpG island of at least one selected from the group consisting of GSTP1, RASSF1A and RARb contained in the serum in the step (a) and the amount of tumor marker in the serum is an abnormal amount in the step (b).

### BRIEF DESCRIPTION OF DRAWINGS

Fig. 1 is a graph showing the amplification curve of real-time PCR with PITPNM1 primer set in Example 1.
Fig. 2 is a graph showing the amplification curve of real-time PCR with GSTP1 primer set in Example 1.
Fig. 3 is a graph showing the amplification curve of real-time PCR with RASSF1A primer set in Example 1.
Fig. 4 is a graph showing the amplification curve of real-time PCR with RARb primer set in Example 1.
Fig. 5 is a drawing-substituting photograph showing the result of confirmation by agarose gel electrophoresis of an amplification product by real-time PCR in Example 1.
Fig. 6 is a diagram showing the result of determining breast cancer metastasis based on the amounts of CA15-3 and CEA and the presence or absence of methylation in the CpG island of each of GSTP1, RASSF1A and RARb in Example 2.

### MODE FOR CARRYING OUT THE INVENTION

### (Method for Determining Breast Cancer Metastasis)

The method for determining breast cancer metastasis of the first embodiment of the present invention includes the steps of obtaining serum from a patient who has undergone surgery to remove breast cancer, detecting methylation in CpG island of each of GSTP1, RASSF1A and RARb contained in the serum obtained in the step of obtaining, and determining that the breast cancer has metastasized, when methylation is detected in the CpG island of at least one selected from the group consisting of GSTP1, RASSF1A and RARb in the step of detecting (also referred to as "determination method 1"). According to the determination method 1, the presence or absence of breast cancer metastasis in a patient who has undergone surgery to remove breast cancer can be determined.

In the determination method 1, a patient who has undergone surgery to remove breast cancer is not particularly limited, and a patient after 3 days or more from the surgery to remove breast cancer is preferable. This is because, after 3 days or more from the surgery, DNA in the blood that has been present before the surgery is degraded, and therefore, an influence on the determination result given by DNA from removed breast cancer can be suppressed.

In the determination method 1, a method for obtaining serum from a patient is not particularly limited, and a known method may be used. For example, the blood collected from a patient is put in a tube and allowed to stand for a while, thereby being separated into serum and clot. The sample after separation into serum and clot is further centrifuged, thereby being completely separated into serum and clot. The supernatant obtained after centrifugation can be thereby obtained as serum.

The GSTP1 described above is glutathione S-transferase pi 1 gene. The nucleotide sequence of GSTP1 is shown in Genebank Accession Number: NC_000011.

In addition, the RASSF1A described above is Ras-association domain family 1A gene. The nucleotide sequence of RASSF1A is shown in Genebank Accession Number: XM_011780.

Furthermore, the RARb described above is 2-retinoic acid receptor β gene. The nucleotide sequence of RARb is shown in Genebank Accession Number: S82362.

In the present embodiment, methylation in CpG island of each of GSTP1, RASSF1A and RARb contained in serum can be detected by using a known method. The known method includes a methylation-specific PCR method, a bisulfite sequencing method, and the like. In these methods, a treatment that converts unmethylated cytosine in DNA to be analyzed into a base other than cytosine using an unmethylated cytosine conversion agent (unmethylated cytosine conversion treatment) is performed.

In the methylation-specific PCR method, a primer set which becomes to allow nucleic acid amplification by PCR method, when cytosine in DNA to be analyzed is not converted to uracil is used. In PCR with this primer set, when a nucleic acid amplification product is detected, cytosine in DNA to be analyzed is found to be methylated cytosine. Examples of the primer set include a primer set comprising a first primer which anneals to a converted nucleic acid comprising a nucleotide sequence in which cytosine in other than a CpG site in a nucleotide sequence containing CpG island is converted into another base (uracil, thymine, or the like) and a second primer which anneals to a complementary strand of the converted nucleic acid, and the like. In the present specification, "anneals to (a converted nucleic acid or complementary strand)" refers to binding via a hydrogen bond to a converted nucleic acid or complementary strand under a reaction temperature adopted in the annealing step during methylation-specific PCR. In addition, in the present specification, the term "hybridize" set forth below is also synonymous.

In addition, in the bisulfite sequencing method, methylation of DNA to be analyzed can be analyzed by determining a nucleotide sequence of the DNA to be analyzed after the unmethylated cytosine conversion treatment.

The unmethylated cytosine conversion treatment is carried out by, for example, a known method. The method is not particularly limited. Examples of a method for carrying out the unmethylated cytosine conversion treatment include a method using an unmethylated cytosine conversion agent, and the like. Examples of the unmethylated cytosine conversion agent include bisulfite such as sodium bisulfite. In the unmethylated cytosine conversion treatment, for example, when bisulfite is used as an unmethylated cytosine conversion agent, unmethylated cytosine is converted into uracil. In the unmethylated cytosine conversion treatment using bisulfite, the concentration of bisulfite is not particularly limited as long as the unmethylated cytosine of DNA contained in serum can be sufficiently converted. The more specific concentration of bisulfite is 1 M or more, preferably 1 to 15 M, and more preferably 3 to 10 M, from the viewpoint of sufficiently converting the unmethylated cytosine of DNA contained in serum. For example, when sodium bisulfite is added to serum so as to have a final concentration of 4 M, conversion of the unmethylated cytosine into uracil can be carried out by incubating at 50° to 80°C for 10 to 90 minutes. In addition, when bisulfite is used at low concentration, the time and temperature may be properly changed to the time and temperature where the unmethylated cytosine can be sufficiently converted.

A concrete primer set for detecting methylation of the CpG island of GSTP1 by using a methylation-specific PCR method includes those containing each primer shown in the following SEQ ID NO: 1 and SEQ ID NO: 2. The primer shown in SEQ ID NO: 1 is a sense primer (GSTP1 sense primer). The primer shown in SEQ ID NO: 2 is an antisense primer (GSTP1 antisense primer).

### 5'-TTCGCGGGATTTTTTAGAAGAGC-3' (SEQ ID NO: 1) 5'-CACTAATAACGAAAACTACGACGACG-3' (SEQ ID NO: 2)

The nucleotide sequence of DNA containing a promoter region of GSTP1, which is subjected to nucleic acid amplification by each primer shown in SEQ ID NO: 1 and SEQ ID NO: 2, is shown in SEQ ID NO: 13.

5'-TCCGCGGACCCTCCAGAAGAGCGGCCGGCGCCGTGACTCAGCACTGGGGC

GGAGCGGGGCGGGACCACCCTTATAAGGCTCGGAGGCCGCGAGGCCTTCGCTG

GAGTTTCGCCGCCGCAGTCTTCGCCACCAGTG-3' (SEQ ID NO: 13)

The primer shown in SEQ ID NO: 1 hybridizes with a nucleic acid comprising a nucleotide sequence complementary to a nucleotide sequence in which cytosine in other than a CpG site has been converted into uracil or thymine in the nucleotide sequence in the range of 1st to 23rd of SEQ ID NO: 13. The primer shown in SEQ ID NO: 2 hybridizes with a nucleic acid comprising a nucleotide sequence in which cytosine in other than a CpG site has been converted into uracil or thymine in the nucleotide sequence in the range of 111th to 136th of SEQ ID NO: 13.

A concrete primer set for detecting methylation of the CpG island of RASSF1A includes a primer set containing each primer shown in the following SEQ ID NO: 3 and SEQ ID NO: 4. The primer shown in SEQ ID NO: 3 is a sense primer (RASSF1A sense primer). The primer shown in SEQ ID NO: 4 is an antisense primer (RASSF1A antisense primer).

### 5'-ATAGTTTTTGTATTTAGGTTTTTATTGCGC-3' (SEQ ID NO: 3) 5'-ACCCGTACTTCGCTAACTTTAAACG-3' (SEQ ID NO: 4)

The nucleotide sequence of DNA containing a promoter region of RASSF1A, which is subjected to nucleic acid amplification by each primer shown in SEQ ID NO: 3 and SEQ ID NO: 4, is shown in SEQ ID NO: 14.

5'-ACAGTCCCTGCACCCAGGTTTCCATTGCGCGGCTCTCCTCAGCTCCTTCCCGC
CGCCCAGTCTGGATCCTGGGGGAGGCGCTGAAGTCGGGGCCCGCCCTGTGGCC
CCGCCCGGCCCGCGCTTGCTAGCGCCCAAAGCCAGCGAAGCACGGGC-3'    (SEQ
ID NO: 14)

The primer shown in SEQ ID NO: 3 hybridizes with a nucleic acid comprising a nucleotide sequence complementary to a nucleotide sequence in which cytosine in other than a CpG site has been converted into uracil or thymine in the nucleotide sequence in the range of 1st to 30th of SEQ ID NO: 14. The primer shown in SEQ ID NO: 4 hybridizes with a nucleic acid comprising a nucleotide sequence in which cytosine in other than a CpG site has been converted into uracil or thymine in the nucleotide sequence in the range of 129th to 153rd of SEQ ID NO: 14.

A concrete primer set for detecting methylation of the CpG island of RARb includes a primer set containing each primer shown in the following SEQ ID NO: 5 and SEQ ID NO: 6. The primer shown in SEQ ID NO: 5 is a sense primer (RARb sense primer). The primer shown in SEQ ID NO: 6 is an antisense primer (RARb antisense primer).
5'-GAATATCGTTTTTTAAGTTAAGTCGTC-3' (SEQ ID NO: 5)
5'-GAAACGCTACTCCTAACTCACG-3' (SEQ ID NO: 6)

The nucleotide sequence of DNA containing a promoter region of RARb, which is subjected to nucleic acid amplification by each primer shown in SEQ ID NO: 5 and SEQ ID NO: 6, is shown in SEQ ID NO: 15.

5'-GAACACCGTTTTCCAAGCTAAGCCGCCGCAAATAAAAAGGCGTAAAGGGAGAG

AAGTTGGTGCTCAACGTGAGCCAGGAGCAGCGTCCC-3' (SEQ ID NO: 15)

The primer shown in SEQ ID NO: 5 hybridizes with a nucleic acid comprising a nucleotide sequence complementary to a nucleotide sequence in which cytosine in other than a CpG site has been converted into uracil or thymine in the nucleotide sequence in the range of 1st to 27th of SEQ ID NO: 15. The primer shown in SEQ ID NO: 6 hybridizes with a nucleic acid comprising a nucleotide sequence in which cytosine in other than a CpG site has been converted into uracil or thymine in the nucleotide sequence in the range of 68th to 89th of SEQ ID NO: 15.

The primer set for detecting methylation of CpG island of each of GSTP1, RASSF1A and RARb can be appropriately designed and is not limited to the primer sets described above.

In addition, in the determination method 1, it is preferred to confirm whether or not the unmethylated cytosine conversion treatment described above is properly carried out. A known method may be used for this confirmation, and the method is not particularly limited. For example, whether or not the unmethylated cytosine conversion treatment is properly carried out can be confirmed by:
(1) identifying a predetermined nucleotide sequence in a region containing unmethylated cytosine in DNA which is to be subjected to analysis for confirming whether or not an unmethylated cytosine conversion treatment is properly carried out;
(2) performing PCR with a control primer set having a primer capable of hybridizing with a nucleic acid comprising the predetermined nucleotide sequence after an unmethylated cytosine conversion treatment, in other words, a primer that anneals to a converted nucleic acid in which all unmethylated cytosines in the DNA have been converted into other bases in the annealing step during PCR or a complementary strand thereof; and
(3) detecting an amplification product by PCR.
   Here, when an amplification product is detected in the above (3), it is an indicator that the unmethylated cytosine conversion treatment is properly carried out. On the contrary, when an amplification product is not detected in the above (3), it is an indicator that the unmethylated cytosine conversion treatment is not properly carried out. In other words, when unmethylated cytosine in the predetermined nucleotide sequence is converted by the unmethylated cytosine conversion treatment, nucleic acid amplification of PCR with a control primer set is possible, and thus the amplification product is detected. On the contrary, when unmethylated cytosine in the identified nucleotide sequence is not converted, nucleic acid amplification of PCR with a control primer set is impossible, and thus the amplification product is not detected.
   Therefore, based on the detection result of the amplification product by PCR with the control primer set, whether or not the unmethylated cytosine conversion treatment is properly carried out can be confirmed.

A concrete control primer set includes a control primer set containing each primer shown in the following SEQ ID NO: 7 and SEQ ID NO: 8. The primer shown in SEQ ID NO: 7 is a sense primer. The primer shown in SEQ ID NO: 8 is an antisense primer.
5'-GGGATATTAAGTGGAGTTATTTTGGTTTTAGTT-3' (SEQ ID NO: 7)
5'-CCCTCCCAACATCCTTCCTAA-3' (SEQ ID NO: 8)

The nucleotide sequence that is subjected to nucleic acid amplification by each primer shown in SEQ ID NO: 7 and SEQ ID NO: 8 is a nucleotide sequence of DNA containing a promoter region of phosphatidylinositol transfer protein (PITPNM1). The nucleotide sequence of DNA containing a promoter region of PITPNM1, which is subjected to nucleic acid amplification, is shown in SEQ ID NO: 16.

5'-GGGATACCAAGTGGAGCCACTCTGGCCTCAGCCTGAGGAGGGCTCACCCCCT

GGGAAGAGGACGGAGTGGCTGCCTAGGCGCGTGGAGAGCCGGCGAGGGCCAG

GGGTCCAGGAAGGATGCTGGGAGGG-3' (SEQ ID NO: 16)

The primer shown in SEQ ID NO: 7 hybridizes with a nucleic acid comprising a nucleotide sequence complementary to a nucleotide sequence in which cytosine has been converted into uracil or thymine in the nucleotide sequence in the range of 1st to 33rd of SEQ ID NO: 16. The primer shown in SEQ ID NO: 8 hybridizes with a nucleic acid comprising a nucleotide sequence in which cytosine has been converted into uracil or thymine in the nucleotide sequence in the range of 109th to 129th of SEQ ID NO: 16. Here, the cytosines in PITPNM1 shown in SEQ ID NO: 16 are all previously confirmed to be unmethylated cytosines by bisulfite sequencing of each of DNAs extracted from a breast cancer tissue and a normal mammary gland.

The control primer set can be appropriately designed and is not limited to the primer set described above.

In the determination step of the determination method 1, it is determined that breast cancer has metastasized when methylation is detected in the CpG island of at least one selected from the group consisting of GSTP1, RASSF1A and RARb in the step of detecting. It is equivalent to determination such that breast cancer has not metastasized when all CpG islands of GSTP1, RASSF1A and RARb are not methylated. By this determination, a patient whose breast cancer has metastasized can be easily screened with high sensitivity.

In the determination method 1, when whether or not the unmethylated cytosine conversion treatment described above is properly carried out is confirmed, the credibility of the determination result can be also determined in the determination step. In other words, when the unmethylated cytosine conversion treatment is properly carried out, it can be determined that the credibility of the determination result is high. On the contrary, when the unmethylated cytosine conversion treatment is not properly carried out, it can be determined that the credibility of the determination result is low. The credibility of the determination result is also determined as described above, and thus a patient whose breast cancer has metastasized can be more accurately screened.

In the present specification, "breast cancer metastasis" shows that breast cancer cells reach to a site different from a primary lesion, proliferate again, and secondarily generate the same type of tumor. In addition, "distant metastasis" shows metastasis where cancer metastasizes in the organs and lymph nodes far from a primary lesion. It is known that breast cancer is likely to distantly metastasize in the supraclavicular lymph nodes, lung, bone, liver, brain, and the like.

The method for determining breast cancer metastasis of the other embodiment of the present invention includes the steps of obtaining serum from a patient who has undergone surgery to remove breast cancer, detecting methylation in CpG island of each of GSTP1, RASSF1A and RARb contained in the serum obtained in the step of obtaining, measuring an amount of tumor marker for breast cancer contained in the serum obtained in the step of obtaining, and determining that the breast cancer has metastasized, when methylation is detected in the CpG island of at least one of GSTP1, RASSF1A and RARb in the step of detecting and the amount of the tumor marker obtained in the step of measuring is an abnormal amount (also referred to as "determination method 2").

Each of the step of obtaining and the step of detecting in the determination method 2 is the same as the step of obtaining and the step of detecting in the determination method 1. In addition, among the terms used in the description of the determination method 2, the terms in common with the terms used in the description of the determination method 1 show the same meaning as the terms used in the description of the determination method 1.

Here, examples of the tumor marker for breast cancer include CA15-3 (carbohydrate antigen 15-3), CEA (Carcinoembryonic antigen), BCA225 (breast cancer antigen-225), NCC-ST-439 (national cancer center-ST439), HER2 (Human Epidermal Growth Factor Receptor 2), and the like. Particularly, CA15-3 and CEA are preferable.

As a method for measuring an amount of tumor marker for breast cancer, a known measurement method such as a method for measuring an amount of tumor marker for breast cancer at protein level using an antibody against tumor marker for breast cancer, a method for measuring an amount of tumor marker for breast cancer at nucleic acid level using a primer set, a probe, a polynucleotide and the like based on a nucleic acid encoding tumor marker for breast cancer or a complementary strand thereof may be used, and the method is not particularly limited. In addition, an apparatus and reagent for measuring an amount of tumor marker for breast cancer are generally commercially available. For example, measurement of an amount of CA15-3 can be carried out by using LUMIPULSE (registered trademark) f which is a full automatic chemical luminescence immunoassay apparatus, and LUMIPULSE (registered trademark) CA15-3 which is a CA15-3 measurement reagent, manufactured by FUJIREBIO Inc. In addition, measurement of an amount of CEA can be carried out by using UniCel Dxl 800 Access (registered trademark) which is a fully automated chemical luminescence immunoassay apparatus, and Access (registered trademark) CEA which is a CEA measurement reagent, manufactured by Beckman Coulter, Inc.

It is the same as in the determination method 2 described above that, in the determination step of the determination method 2, it is determined that breast cancer has metastasized when methylation is detected in the CpG island of at least one selected from the group consisting of GSTP1, RASSF1A and RARb.

Here, in the determination step of the determination method 2, it is determined that breast cancer has metastasized also when the amount of the tumor marker obtained in the step of measuring is an abnormal amount. It is equivalent to determination such that breast cancer has not metastasized when the amount of tumor marker is a normal amount. By this determination, a patient whose breast cancer has metastasized can be more accurately screened.

Here, the "abnormal amount" shows that the amount of tumor marker for breast cancer contained in the serum obtained in the step of obtaining is an amount outside the range of the normal amount of tumor marker for breast cancer contained in the serum of a normal subject. On the other hand, the normal amount shows that the amount of tumor marker for breast cancer contained in the serum obtained in the step of obtaining is an amount within the range of the normal amount of tumor marker for breast cancer contained in the serum of a normal subject. The determination of the abnormal amount or the normal amount can be carried out, for example, based on the comparison result of comparing the amount of the tumor marker obtained in the step of measuring with a predetermined threshold. More specifically, when the amount of the tumor marker obtained in the step of measuring exceeds the predetermined threshold, the amount can be determined as an abnormal amount. When the amount of the tumor marker obtained in the step of measuring is not more than the predetermined threshold, the amount can be determined as a normal amount. The predetermined threshold can be set based on the measurement result of the amount of tumor marker for breast cancer contained in the serum of a plurality of normal subjects and a plurality of patients whose breast cancer has metastasized. In addition, when the commercially available apparatuses and reagents for measuring the amount of tumor marker for breast cancer described above are used, the thresholds described in the instruction manual can be also used.

As described above, according to the method for determining breast cancer metastasis of the present invention, since an apparatus used for a conventional imaging test is not used in the determination, the presence or absence of breast cancer in a patient who has undergone surgery to remove breast cancer can be easily determined at low cost without giving the patient an uncomfortable feeling. According to this method for determining breast cancer metastasis, a recurrence by breast cancer metastasis can be also detected in an early stage by periodically determining the presence or absence of breast cancer metastasis, particularly breast cancer distant metastasis, by the determination method, after the surgery to remove breast cancer.

### (Other Application Examples)

By applying the method for determining breast cancer described above, it is possible to carry out a serum evaluation in which whether or not serum is collected from a patient whose breast cancer has metastasized is evaluated, in other words, it is possible to carry out the provision of an indicator of the possibility of breast cancer metastasis, a test for taking the measure of breast cancer metastasis, and the like. These provide information useful for the determination of breast cancer metastasis for a doctor to diagnose, by testing institutes and the like.

The method for evaluating serum of the present invention is, in one aspect, a method including the steps of:
(A) determining the presence or absence of methylation in CpG island of each of GSTP1, RASSF1A and RARb contained in serum collected from a patient who has undergone surgery to remove breast cancer, and
(B) evaluating that the serum is serum collected from a patient whose breast cancer has metastasized, when methylation is detected in the CpG island of at least one selected from the group consisting of GSTP1, RASSF1A and RARb contained in the serum in the step (A) (evaluation method 1). In addition, the method for evaluating serum of the present invention is, in another aspect, a method including the steps of:
   (a) determining the presence or absence of methylation in CpG island of each of GSTP1, RASSF1A and RARb contained in serum collected from a patient who has undergone surgery to remove breast cancer,
   (b) measuring an amount of tumor marker for breast cancer contained in the serum, and
   (c) evaluating the serum as serum collected from a patient whose breast cancer has metastasized, when methylation is detected in the CpG island of at least one selected from the group consisting of GSTP1, RASSF1A and RARb contained in the serum in the step (a) and the amount of tumor marker in the serum is an abnormal amount in the step (b).

Among the terms used in the descriptions of the evaluation method 1 and evaluation method 2, the terms in common with the terms used in the description of the determination method 1 described above show the same meaning as the terms used in the description of the determination method 1.

The step (A) of the evaluation method 1 and the step (a) of the evaluation method 2 can be carried out in the same manner as in the step of detecting of the determination method 1.

The step (B) of the evaluation method 1 is a step that can be carried out in testing institutes and the like. In this step (B), the serum is evaluated as serum collected from a patient whose breast cancer has metastasized, when methylation is detected in the CpG island of at least one selected from the group consisting of GSTP1, RASSF1A and RARb contained in the serum in the step (A).

In the step (B) of the evaluation method 1, for example, whether or not the serum is serum collected from a patient whose breast cancer has metastasized can be evaluated by comparing the result obtained in the step (A) of the evaluation method 1 with the criteria that the serum is serum from a patient whose breast cancer has metastasized when serum contains DNA in which methylation is present in the CpG island of at least one selected from the group consisting of GSTP1, RASSF1A and RARb. The comparative result thus obtained can be also provided to a person who determines breast cancer metastasis as an indicator of the possibility of breast cancer metastasis.
In addition, from the comparative result, the data for taking the measure of breast cancer metastasis whether or not a patient has breast cancer metastasis can be obtained.

On the other hand, the step (b) of the evaluation method 2 can be carried out by operating in the same manner as in the step of measuring in the determination method 1. The step (c) of the evaluation method 2 can be carried out in testing institutes and the like. In this step (c), the serum is evaluated as serum collected from a patient whose breast cancer has metastasized, when methylation is detected in the CpG island of at least one selected from the group consisting of GSTP1, RASSF1A and RARb contained in the serum in the step (a) and the amount of tumor marker in the serum is an abnormal amount in the step (b).

In the step (c) of the evaluation method 2, whether or not the serum is serum collected from a patient whose breast cancer has metastasized can be evaluated by comparing the result obtained in the steps (a) and (b) with the criteria that the serum is serum from a patient whose breast cancer has metastasized when serum contains DNA in which methylation is present in the CpG island of at least one selected from the group consisting of GSTP1, RASSF1A and RARb and the amount of tumor marker is an abnormal amount. The resulting comparative result can be also provided to a person who determines breast cancer metastasis as an indicator of the possibility of breast cancer metastasis.
In addition, from the comparative result, the data for taking the measure of breast cancer metastasis whether or not a patient has breast cancer metastasis can be obtained.

According to the method for evaluating serum of the present invention, since an apparatus used for a conventional imaging test is not used as well as the method for determining breast cancer metastasis described above, an indicator for determining the presence or absence of breast cancer metastasis in a patient who has undergone surgery to remove breast cancer can be obtained easily at low cost without giving the patient an uncomfortable feeling in the evaluation.

Hereinafter, the present invention will be described in detail by way of examples, but the present invention is not limited thereto.

### EXAMPLES

### (Example 1)

### (1) Obtainment of Serum

About 6 ml of blood was collected from each of 6 patients who had diagnosis of recurrence due to breast cancer distant metastasis using a blood collection tube containing a serum separation agent (trade name: Venoject II vacuum blood collection tube, manufactured by TERUMO CORPORATION). After 30 minutes from blood collection, the blood collection tube was subjected to centrifugation at 1200 G for 10 minutes. After centrifugation, the supernatant was obtained as serum.

### (2) Unmethylated Cytosine Conversion Treatment of DNA Contained in Serum

To 300 µl of serum, 300 µl of an aqueous solution of 8 M guanidine hydrochloride and 20 µl of an aqueous solution of 20 mg/ml proteinase K (manufactured by Sigma-Aldrich Inc.) were added and mixed, and the resulting mixture was kept at 50°C for 1 hour. Next, 20 µl of an aqueous solution of 10 M sodium hydroxide was added to the mixture after keeping the heat and mixed, and the resulting mixture was kept at room temperature for 10 minutes. Thereafter, a sodium bisulfite treatment was carried out by adding 600 µl of an aqueous solution of 10 M sodium bisulfite to the mixture after keeping the heat, thereby mixing them, and then keeping the resulting mixture at 80°C for 40 minutes. The DNA contained in the sodium bisulfite-treated sample was purified with a purification kit (trade name: OIAquick PCR Purification Kit, manufactured by QIAGEN), and the purified product was recovered by eluting with 50 µl of distilled water. To 50 µl of the recovered DNA solution, 5.5 µl of a 3 M sodium hydroxide solution was added and mixed, and the mixture was kept at room temperature. Immediately after 5 minutes, the resulting mixture was purified with a gel filtration medium (trade name: sephacryl S-300, manufactured by GE Healthcare) to remove sodium hydroxide. The recovered DNA solution was defined as a solution for detecting methylation.

### (2) Detection of Methylated Cytosine by Methylation-Specific PCR Method

Using the solution for detecting methylation, real-time PCR was performed under the following conditions.

### (2-1) Real-time PCR with PITPNM1 Primer Set

Real-time PCR was performed using PITPNM1 primer set as a control primer set for confirming whether or not an unmethylated cytosine conversion treatment was sufficiently carried out. PITPNM1 primer set contains the primer shown in SEQ ID NO: 7 and the primer shown in SEQ ID NO: 8 as described above, and a fluorescently labeled probe shown in the following SEQ ID NO: 9. The fluorescently labeled probe was used in the detection of an amplification product by a fluorescent detection method by using real-time PCR.

FAM-ATTTTTTGGGAAGAGGATGGAGTGGTTGTTTAGG-DarkQuencher (SEQ ID NO: 9)
FAM (5-carboxy-fluorescein) is a fluorescent dye, and DarkQuencher (registered trademark of Dark Quencher (Epoch Biosciences)) shows a quencher (excitation energy absorber). Also, the fluorescent probe shown in SEQ ID NO: 9 hybridizes with a portion comprising a nucleotide sequence in which cytosine has been converted into uracil or thymine in the nucleotide sequence in the range of 46th to 80th of SEQ ID NO: 16.

The composition of the reaction solution for real-time PCR with PITPNM1 primer set is shown below.

**[Table 1]**

| Reagent for Real-Time PCR | |
|---|---|
| (Trade Name: FastStart TaqMan Probe Master (ROX), | 10 µl |
| Manufactured by Roche Diagnostics K.K.) | |
| Primer of SEQ ID NO: 7 (100 µM) | 0.24 µl |
| Primer of SEQ ID NO: 8 (100 µM) | 0.24 µl |
| Probe of SEQ ID NO: 9 (10 µM) | 0.3 µl |
| Solution for Detecting Methylation | 9 µl |
| dH₂O | 0.22 µl |
| Total | 20 µl |

The reaction conditions of real-time PCR with PITPNM1 primer set are shown below.

**[Table 2]**

| | | |
|---|---|---|
| 95°C | 9 minutes and 30 seconds | 1 cycle |
| 95°C | 30 seconds | 45 cycles |
| 60°C | 30 seconds | |
| 72°C | 30 seconds | |
| 4°C | | Storage |

### (2-2) Real-time PCR with GSTP1 Primer Set

GSTP1 primer set for detecting methylation of the CpG island of GSTP1 contains the primer shown in SEQ ID NO: 1, the primer shown in SEQ ID NO: 2, and a fluorescently labeled probe shown in the following SEQ ID NO: 10.

FAM-ATAAGGTTCGGAGGTCGCGAGGTTTTCGT-DarkQuencher (SEQ ID NO: 10)
The fluorescently labeled probe shown in SEQ ID NO: 10 hybridizes with a portion comprising a nucleotide sequence in which cytosine in other than a CpG site is converted into uracil or thymine in the nucleotide sequence in the range of 74th to 103rd of SEQ ID NO: 13.

The composition of the reaction solution of real-time PCR with GSTP1 primer set is shown below.

**[Table 3]**

| Reagent for Real-Time PCR | |
|---|---|
| (Trade Name: FastStart TaqMan Probe Master (ROX), | 10 µl |
| Manufactured by Roche Diagnostics K.K.) | |
| Primer of SEQ ID NO: 1 (100 µM) | 0.18 µl |
| Primer of SEQ ID NO: 2 (100 µM) | 0.18 µl |
| Probe of SEQ ID NO: 10 (10 µM) | 0.5 µl |
| Solution for Detecting Methylation | 9 µl |
| dH₂O | 0.14 µl |
| Total | 20 µl |

The reaction conditions of real-time PCR with GSTP1 primer set are the same as those with PITPNM1 primer set.

### (2-3) Real-time PCR with RASSF1A Primer Set

RASSF1A primer set for detecting methylation of the CpG island of RASSF1A contains the primer shown in SEQ ID NO: 3, the primer shown in SEQ ID NO: 4, and a fluorescently labeled probe shown in the following SEQ ID NO: 11.

FAM-TTGAAGTCGGGGTTCGTTTTGTGGTTTCGT-DarkQuencher (SEQ ID NO: 11)
The probe shown in SEQ ID NO: 11 hybridizes with a portion comprising a nucleotide sequence in which cytosine in other than a CpG site has been converted into uracil or thymine in the nucleotide sequence in the range of 81 st to 111th of SEQ ID NO: 14.

The composition of the reaction solution of real-time PCR with RASSF1A primer set is shown below.

**[Table 4]**

| Reagent for Real-Time PCR | |
|---|---|
| (Trade Name: FastStart TaqMan Probe Master (ROX), | |
| | 10 µl |
| Manufactured by Roche Diagnostics K.K.) | |
| Primer of SEQ ID NO: 3 (100 µM) | 0.18 µl |
| Primer of SEQ ID NO: 4 (100 µM) | 0.18 µl |
| Probe of SEQ ID NO: 11 (10 µM) | 0.3 µl |
| Solution for Detecting Methylation | 9 µl |
| dH₂O | 0.34 µl |
| Total | 20 µl |

The reaction conditions of real-time PCR with RASSF1A primer set are the same as those with PITPNM1 primer set.

### (2-4) Real-time PCR with RARb Primer Set

RARb primer set for detecting methylation of the CpG island of RARb contains the primer shown in SEQ ID NO: 5, the primer shown in SEQ ID NO: 6, and a fluorescently labeled probe shown in the following SEQ ID NO: 12.

FAM-AGGCGTAAAGGGAGAGAAGTTGGTGTTTA-DarkQuencher (SEQ ID NO: 12)
The probe shown in SEQ ID NO: 12 hybridizes with a portion comprising a nucleotide sequence in which cytosine in other than a CpG site is converted into uracil or thymine in the nucleotide sequence in the range of 38th to 67th of SEQ ID NO: 15.

The composition of the reaction solution of real-time PCR with RARb primer set is shown below.

**[Table 5]**

| Reagent for Real-Time PCR | |
|---|---|
| (Trade Name: FastStart TaqMan Probe Master (ROX), | |
| | 10 µl |
| Manufactured by Roche Diagnostics K.K.) | |
| Primer of SEQ ID NO: 5 (100 µM) | 0.18 µl |
| Primer of SEQ ID NO: 6 (100 µM) | 0.18 µl |
| Probe of SEQ ID NO: 12 (10 µM) | 0.5 µl |
| Solution for Detecting Methylation | 9 µl |
| dH₂O | 0.14 µl |
| Total | 20 µl |

The reaction conditions of real-time PCR with RARb primer set are the same as those with PITPNM1 primer set.

### (3) Determination of Breast Cancer Metastasis and Evaluation of Serum

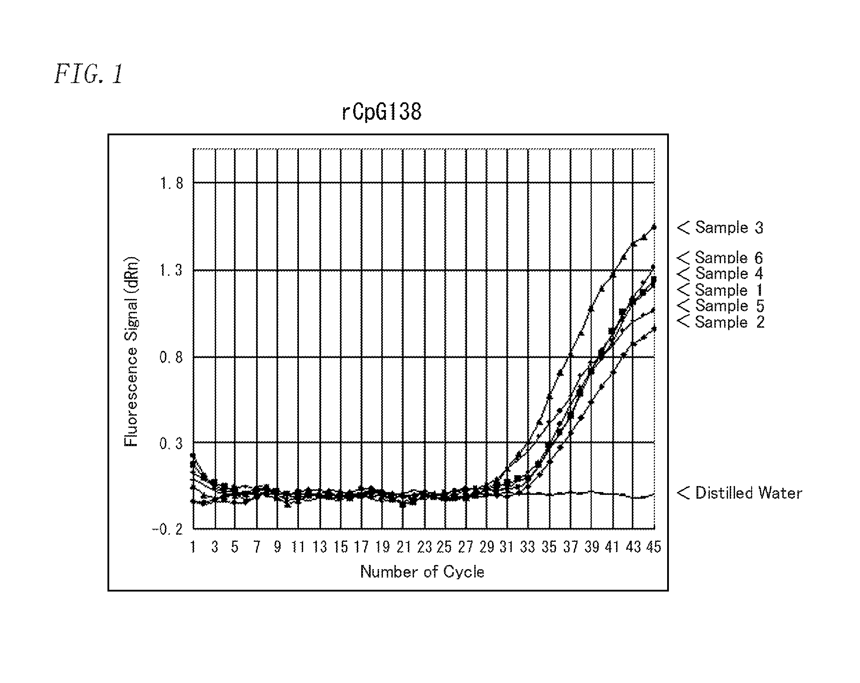

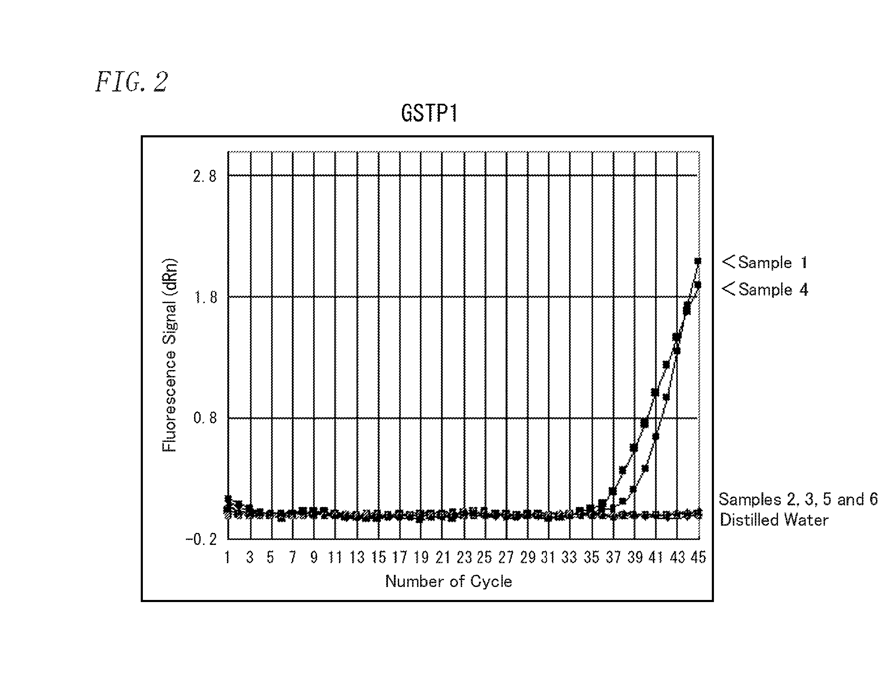

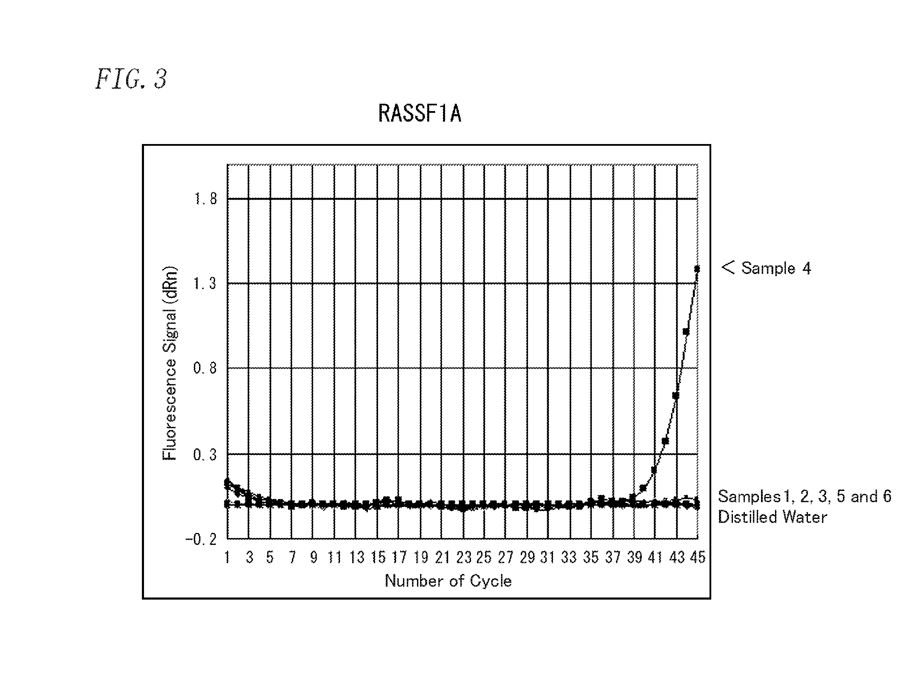

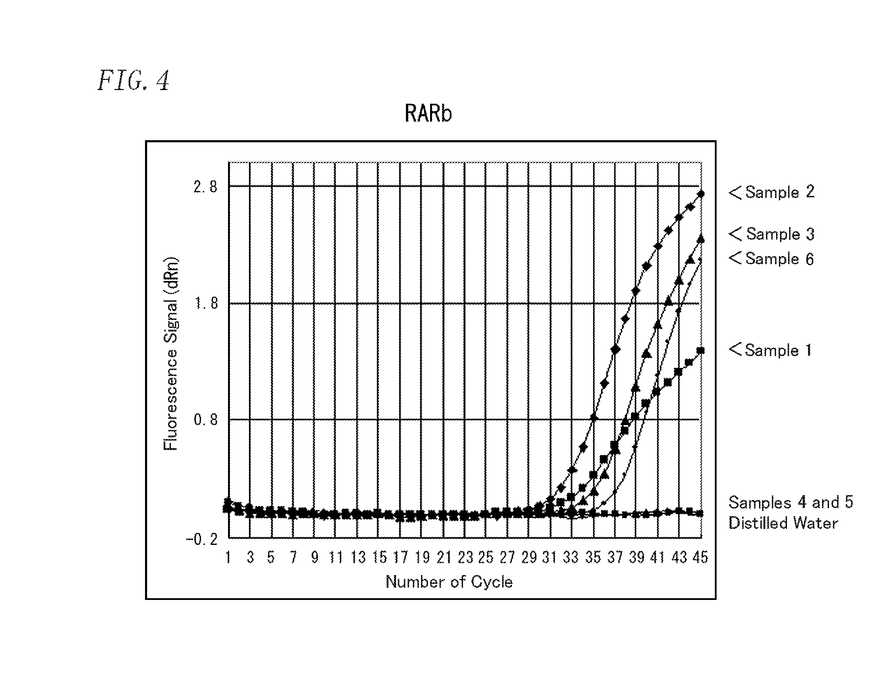

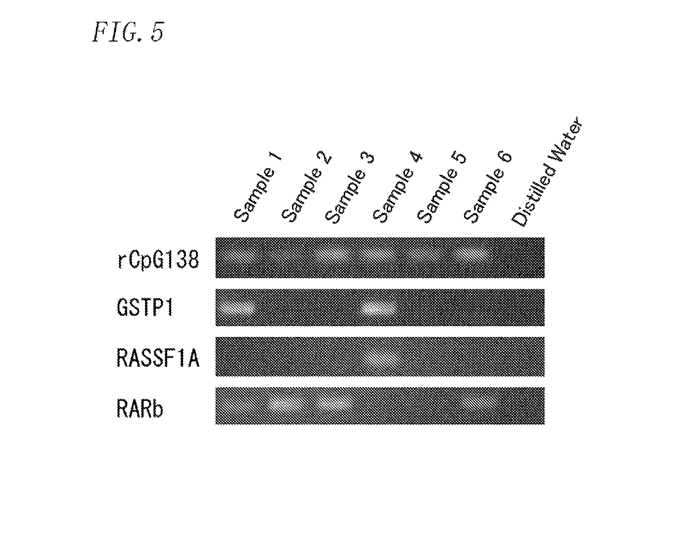

The results of the amplification curves by the real-time PCR described above are shown in Figs. 1 to 4. Here, Fig. 1 is a graph showing the amplification curve of real-time PCR with PITPNM1 primer set in Example 1. Fig. 2 is a graph showing the amplification curve of real-time PCR with GSTP1 primer set in Example 1. Fig. 3 is a graph showing the amplification curve of real-time PCR with RASSF1A primer set in Example 1. Fig. 4 is a graph showing the amplification curve of real-time PCR with RARb primer set in Example 1. In addition, Fig. 5 is a drawing-substituting photograph showing the result of confirmation by agarose gel electrophoresis of an amplification product by real-time PCR in Example 1. "Distilled water" in Figs. 1 to 5 is the result of performing real-time PCR using distilled water in place of a solution for detecting methylation.

It can be seen from the results shown in Fig. 1 and Fig. 5 that amplification products are confirmed in all 6 samples, except for distilled water, by real-time PCR with PITPNM1 primer set. It can be seen from the above result that the unmethylated cytosine conversion treatment of all 6 samples were properly carried out.

Also, it can be seen from the results shown in Fig. 2 and Fig. 5 that amplification products are confirmed in sample 1 and sample 4 by real-time PCR with GSTP1 primer set. It can be seen from the above result that the CpG islands of GSTP1 in sample 1 and sample 4 are methylated.

Also, it can be seen from the results shown in Fig. 3 and Fig. 5 that an amplification product is confirmed in sample 4 by real-time PCR with RASSF1A primer set. It can be seen from the above result that the CpG island of RASSF1A in sample 4 is methylated.

Also, it can be seen from the results shown in Fig. 4 and Fig. 5 that amplification products are confirmed in sample 1, sample 2, sample 3, and sample 6 by real-time PCR with RARb primer set. It can be seen from the above result that the CpG islands of RARb in sample 1, sample 2, sample 3 and sample 6 are methylated.

It can be seen from the above results that the methylation is detected in the CpG island of at least one of GSTP1, RASSF1A and RARb in 5 serum samples among 6 serum samples obtained from 6 patients who had diagnosis of recurrence by breast cancer distant metastasis. It can be seen from the above result that, in this Example, among 6 samples that had diagnosis of recurrence by distant metastasis in breast cancer, 5 samples can be determined to have breast cancer metastasis.

It can be seen from these results that breast cancer distant metastasis can be determined based on the presence of the methylation in the CpG island of at least one of GSTP1, RASSF1A and RARb contained in serum.

In addition, it can be seen that whether or not the serum is serum collected from a patient whose breast cancer has metastasized can be evaluated by detecting the methylation in the CpG island of at least one of GSTP1, RASSF1A and RARb contained in serum, based on the criteria that the serum is serum from a patient whose breast cancer has metastasized when the methylation in the CpG island of at least one of GSTP1, RASSF1A and RARb contained in serum is detected.

### (Example 2)

### (1) Obtainment of Serum

Serum samples were obtained from 34 patients who had diagnosis of recurrence by breast cancer distant metastasis in the same manner as in Example 1.

### (2) Measurement of CA15-3 and CEA

Amounts of tumor markers CA15-3 and CEA, were measured for each serum obtained. An amount of CA15-3 was measured by using LUMIPULSE (registered trademark) f and LUMIPULSE (registered trademark) CA15-3 manufactured by FUJIREBIO Inc. In addition, an amount of CEA was measured by using UniCel Dxl 800 Access (registered trademark) and Access (registered trademark) CEA manufactured by Beckman Coulter, Inc. Here, a sample at a concentration of CA15-3 in the serum above 30 U/ml was determined as a sample positive for breast cancer metastasis. In addition, a sample at a concentration of CEA in the serum above 5 ng/ml was determined as positive for breast cancer metastasis.

### (3) Detection of Methylation in CpG Island of Each of GSTP1, RASSF1A and RARb

For each serum obtained, the presence or absence of methylation in CpG island of each of GSTP1, RASSF1A and RARb was examined in the same manner as in Example 1. Here, a sample where methylation was detected in the CpG island of at least one of GSTP1, RASSF1A and RARb was determined as a sample positive for breast cancer metastasis.

### (4) Determination of Breast Cancer Metastasis and Evaluation of Serum

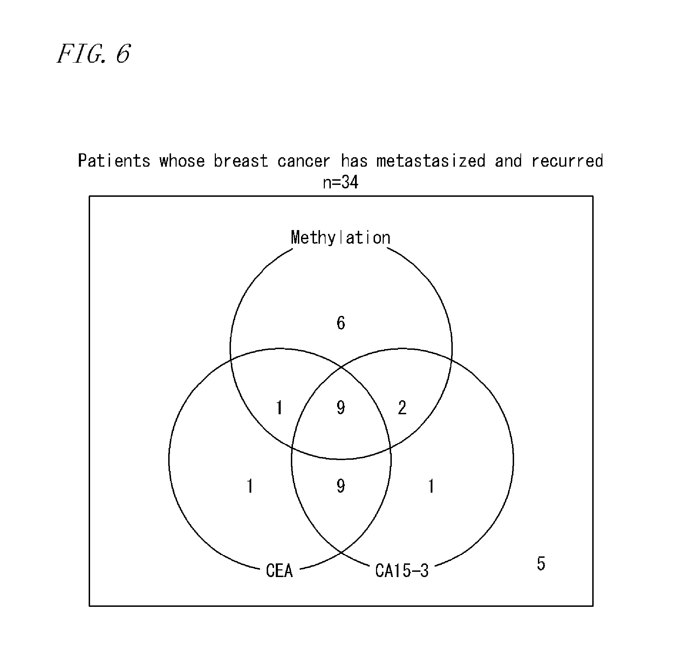

The diagram showing the result of determining breast cancer metastasis based on the amounts of CA15-3 and CEA and the presence or absence of the methylation of the CpG islands of GSTP1, RASSF1A and RARb in Example 2 is shown in Fig. 6. In the diagram, each number surrounded by a frame shows the number of sample which was determined as positive for breast cancer metastasis using the amount of CEA as an indicator, the number of sample which was determined as positive for breast cancer metastasis using the amount of CA15-3 as an indicator, or the number of sample which was determined as positive for breast cancer metastasis using the methylation of the CpG island of at least one of GSTP1, RASSF1A and RARb as an indicator. In addition, in the diagram, each number in the part where 2 or more frames overlap shows the number of sample which was determined as positive for breast cancer metastasis in common by corresponding 2 or more indicators.

From the result shown in Fig. 6, in the conventional determination of breast cancer metastasis using both the amount of CA15-3 and the amount of CEA as an indicator, 11 samples out of 34 samples were determined as negative for breast cancer metastasis. In addition, when the determination sensitivity is calculated by the sample number of positive determination/the total sample number, it can be seen that the determination of breast cancer metastasis using both the amount of CA15-3 and the amount of CEA as an indicator has a sensitivity of only 68% (23 samples of positive determination/total 34 samples).

However, it can be seen that, when the determination using the presence of methylation as an indicator is further added, in addition to the determination of breast cancer metastasis using both the amount of CA15-3 and the amount of CEA as an indicator, 6 samples out of 11 samples, which were determined as negative for breast cancer metastasis by the determination of breast cancer metastasis using each amount of CA15-3 and CEA as an indicator, are determined as positive for breast cancer metastasis, and the sensitivity improves to 85% (29 samples of positive determination/total 34 samples).

In addition, it can be seen that, even by the combination of the determination of breast cancer metastasis using the amount of CA15-3 or the amount of CEA as an indicator with the determination of breast cancer metastasis by the detection of methylation, the sensitivity is about 85% (each 29 samples of positive determination/total 34 samples).

It can be seen from the above results that breast cancer metastasis can be determined with higher sensitivity by carrying out the determination of breast cancer metastasis based on the presence of the methylation in the CpG island of at least one of GSTP1, RASSF1A and RARb, in addition to the determination of breast cancer metastasis by conventional tumor marker for breast cancer.

In addition, it can be seen that, by determining an amount of tumor marker for breast cancer contained in serum and detecting methylation in the CpG island of at least one of GSTP1, RASSF1A and RARb in the serum, whether or not the serum is serum collected from a patient whose breast cancer has metastasized can be evaluated based on the criteria that the serum is serum from a patient whose breast cancer has metastasized when methylation in the CpG island of at least one of GSTP1, RASSF1A and RARb in the serum is detected and the amount of tumor marker in serum is an abnormal amount.

### Sequence Listing Free Text

SEQ ID NO: 1 is a sequence of GSTP1 sense primer.
SEQ ID NO: 2 is a sequence of GSTP1 antisense primer.
SEQ ID NO: 3 is a sequence of RASSF1A sense primer.
SEQ ID NO: 4 is a sequence of RASSF1A antisense primer.
SEQ ID NO: 5 is a sequence of RARb sense primer.
SEQ ID NO: 6 is a sequence of RARb antisense primer.
SEQ ID NO: 7 is a sequence of PITPNM1 sense primer.
SEQ ID NO: 8 is a sequence of PITPNM1 antisense primer.
SEQ ID NO: 9 is a sequence of probe.
SEQ ID NO: 10 is a sequence of probe.
SEQ ID NO: 11 is a sequence of probe.
SEQ ID NO: 12 is a sequence of probe.

## Claims

1. A method for determining breast cancer metastasis comprising the steps of:
obtaining serum from a patient who has undergone surgery to remove breast cancer,
detecting methylation in CpG island of each of GSTP1, RASSF1A and RARb contained in the serum obtained in the step of obtaining, and
determining that the breast cancer has metastasized, when methylation is detected in the CpG island of at least one selected from the group consisting of GSTP1, RASSF1A and RARb in the step of detecting.

2. The method according to claim 1, wherein the metastasis is distant metastasis.

3. The method according to claim 1 or 2, wherein the methylation in CpG island of each of GSTP1, RASSF1A and RARb is detected by using a methylation-specific PCR method in the step of detecting.

4. The method according to any one of claims 1 to 3, wherein the methylation-specific PCR method is performed by using a first primer set comprising the primers shown in SEQ ID NO: 1 and SEQ ID NO: 2,
a second primer set comprising the primers shown in SEQ ID NO: 3 and SEQ ID NO: 4, and
a third primer set comprising the primers shown in SEQ ID NO: 5 and SEQ ID NO: 6.

5. A method for determining breast cancer metastasis comprising the steps of:
obtaining serum from a patient who has undergone surgery to remove breast cancer,
detecting methylation in CpG island of each of GSTP1, RASSF1A and RARb contained in the serum obtained in the step of obtaining,
measuring an amount of tumor marker for breast cancer contained in the serum obtained in the step of obtaining, and
determining that the breast cancer has metastasized, when methylation is detected in the CpG island of at least one selected from the group consisting of GSTP1, RASSF1A and RARb in the step of detecting and the amount of the tumor marker obtained in the step of measuring is an abnormal amount.

6. The method according to claim 5, wherein the tumor marker for breast cancer is CEA and/or CA15-3.

7. A method for evaluating serum comprising the steps of:
(A) determining the presence or absence of methylation in CpG island of each of GSTP1, RASSF1A and RARb contained in serum collected from a patient who has undergone surgery to remove breast cancer, and
(B) evaluating the serum as serum collected from a patient whose breast cancer has metastasized, when methylation is detected in the CpG island of at least one selected from the group consisting of GSTP1, RASSF1A and RARb contained in the serum in the step (A).

8. A method for evaluating serum comprising the steps of:
(a) determining the presence or absence of methylation in CpG island of each of GSTP1, RASSF1A and RARb contained in serum collected from a patient who has undergone surgery to remove breast cancer,
(b) measuring an amount of tumor marker for breast cancer contained in the serum, and
(c) evaluating the serum as serum collected from a patient whose breast cancer has metastasized, when methylation is detected in the CpG island of at least one selected from the group consisting of GSTP1, RASSF1A and RARb contained in the serum in the step (a) and the amount of tumor marker in the serum is an abnormal amount in the step (b).
